(19) Europäisches Patentamt
European Patent Office
Office européen des brevets

(11) Numéro de publication: **0 037 354**
**B1**

(12) # FASCICULE DE BREVET EUROPEEN

(45) Date de publication du fascicule du brevet: **18.05.83**

(51) Int. Cl.³: **C 07 C 51/12**, C 07 C 53/08

(21) Numéro de dépôt: **81420048.1**

(22) Date de dépôt: **30.03.81**

(54) **Procédé de préparation de l'acide acétique par carbonylation.**

(30) Priorité: **02.04.80 FR 8007876**

(43) Date de publication de la demande:
**07.10.81 Bulletin 81/40**

(45) Mention de la délivrance du brevet:
**18.05.83 Bulletin 83/20**

(84) Etats contractants désignés:
**AT BE CH DE FR GB IT LI LU NL SE**

(56) Documents cités:
**BE - A - 366 710**
**FR - A - 1 045 404**

(73) Titulaire: **RHONE-POULENC CHIMIE DE BASE**
**25, quai Paul Doumer**
**F-92408 Courbevoie (FR)**

(72) Inventeur: **Gauthier-Lafaye, Jean**
**21, rue Duguesclin**
**F-69006 Lyon (FR)**
Inventeur: **Perron, Robert**
**La Pecolière**
**F-69390 Charly (FR)**

(74) Mandataire: **Varniere-Grange, Monique et al,**
**RHONE-POULENC RECHERCHES Centre de Recherches de Saint-Fons Service Brevets B.P. 62**
**F-69190 Saint-Fons (FR)**

**EP 0 037 354 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procede de preparation de l'acide acétique par carbonylation

La présente invention a pour objet un procédé de préparation de l'acide acétique, par carbonylation du méthanol.

Il est bien connu, en particulier, que l'acide acétique peut être produit par carbonylation du méthanol, dans des conditions relativement sévères de pression, en présence de nickel et d'un halogène libre ou combiné.

C'est ainsi qu'il a été proposé, notamment, (Cf Brevet des ETATS UNIS D'AMERIQUE no. 2 729.651) de réaliser la carbonylation du méthanol en présence de complexes du nickel, obtenus par réaction d'halogénures de nickel avec des halogénures d'ammonium (ou de phosphonium) quaternaire, complexes de formule générale:

$$[A_4M]_2NiX_4$$

dans laquelle X représente un atome de brome ou d'iode, M un atome de phosphore ou d'azote, A étant, par exemple, un radical alcoyle inférieur.

Ces complexes peuvent être engagés sous cette forme dans la réaction en cause, ou biel ils peuvent être formés in situ. Toutefois, bien que la pression mise en oeuvre lors de la réaction de carbonylation soit élevée (de l'ordre de 700 atmosphères) l'efficacité du système catalytique, exprimée en terme de productivité horaire, est très faible.

Cette productivité exprimée sout par rapport au volume réactionnel, soit par rapport à la masse de nickel mise en oeuvre, a pu être sensiblement améliorée en engageant dans la réaction en cause d'une part, un halogénure de nickel et d'autre part, un halogénure d'ammonium (ou de phosphonium) quaternaire en quantité supérieure à celle requise par la stoechiométrie de formation des complexes de formule rappelée ci-avant. (Cf. Brevet allemand 933.148). Toutefois, même dans ce dernier cas, les conditions de pression restent sévères.

Plus récemment, il a été proposé de systèmes catalytiques permettant de carbonyler le méthanol dans des conditions moins sévères de pression. Ainsi, la demande de brevet français 2.370,023 décrit la carbonylation du méthanol en présence d'au moins 10 moles d'iodure de méthyle pour 100 moles de méthanol, de nickel, et d'une phosphine libre et/ou complexée avec le nickel, sous une pression inférieure à 70 bars. Néanmoins, l'efficacité d'un tel système, exprimée en terme de productivité horaire reste faible.

Dans le cadre d'un procédé de carbonylation sous faible pression, il a également été souligné (cf. demande de brevet français 2.404.618) que la présence de solvants, tels que des acides carboxyliques ou leurs esters, a un effet favorable sur le déroulement de la réaction de carbonylation. Néanmoins, l'utilisation de tels solvants n'est pas essentielle et l'alcool dont on part peut servir de solvent.

Toutefois, l'interêt industriel de ces techniques récentes est compromis par l'instabilité et le coût des phosphines ou des amines nécessaires à leur mise en oeuvre.

Il a maintenant été trouvé qu'il est possible de préparer des acides carboxyliques, en particulier des acides alcanoïques inférieurs et notamment l'acide acétique par carbonylation d'alcools en présence de nickel et d'au moins un promoteur halogéné avec une productivité remarquable, dans des conditions de pression relativement douces, tout en obviant aux inconvénients précités.

La présente invention a donc pour objet un procédé perfectionné de carbonylation du méthanol en phase liquide à une température supérieure à 120°C et sous une pression totale inférieure à 200 bars en présence d'un quantité efficace de nickel, d'un halogénure d'alkyle ou d'acyle, d'au moins un sel de chrome, dans lequel le chrome est à un degré d'oxydation (p) supérieur à zéro (p>o) et d'un acide carboxylique initialement chargé.

Il a été trouvé de manière inattendue, en particulier dans le cadre de la carbonylation du méthanol en acide acétique, que les résultants ne sont pas satisfaisants si la pression partielle du monoxyde de carbone est trop élevée; ceci est d'autant plus surprenant que de nombreux travaux antérieurs avaient mis en évidence la nécessité de travailler sour des pressions très élevées.

Les recherches qui ont abouti à la présente invention ont permis de faire ressortir le comportement surprenant des sels de chrome, dans lequels le chrome est à un degré d'oxydation (p) supérieur à zéro (p>0) . comme cocatalyseurs de la réaction de carbonylation d'un alcool, en phase liquide et sous une pression totale inférieure à 200 bars. La demanderesse a en effet constaté que les sels et autres dérivés de nombreux métaux (y compris des métaux carbonyles) ne permettent pas de faire réagir dans les conditions précitées le monoxyde de carbone sur un alcool, dans un acide carboxylique, en présence de nickel et d'un promoteur halogéné.

Le procédé selon la présente invention est conduit en phase liquide en présence d'un acide carboxylique, de formule $R^1COOH$, initialement chargé, dans lequel $R^1$ représente un radical alkyle linéaire, remifié ou cyclique renfermant de 1 à 6 atomes de carbone ou un radical $\Phi—C_nH_{2n}—$ dans lequel n est un entier compris entre 1 et 6 ($1 \leqslant n \leqslant 6$). En d'autres termes l'acide carboxylique qui joue en quelque sorte un rôle de solvant n'est pas forcément l'acide carboxylique produit par la réaction de carbonylation. Toutefois, il peut s'avérer préférable que l'acide carboxylique utilisé comme solvant soit

celui produit par la réaction. Néanmoins, l'usage à titre de solvent d'un acide carboxylique plus lourd que l'acide produit, peut faciliter les opérations de séparation.

L'acide carboxylique $R^1COOH$ représente au moins 10 % en volume du milieu réactionnel initial. De préférence, il représente au moins 20 % en volume du milieu réactionnel. Il peut représenter une partie important du milieu réactionnel, notamment dans le cas d'une opération réalisée en continu en injectant le méthanol dans le réacteur de carbonylation.

Le procédé selon l'invention nécessite la présence d'une quantité efficace de nickel, N'importe quelle source de nickel peut être mise en oeuvre dans le cadre du présent procédé. On peut introduire le nickel sous sa forme métallique (nickel RANEY, par exemple) ou sous toute autre forme commode. A titre d'exemples de composés du nickel susceptibles d'être utilisés pour la mise en oeuvre du présent procédé, on peut citer: le carbonate, l'oxyde, l'hydroxyde, les halogenures, en particulier l'iodure, les carboxylates, en particulier l'acétate, de nickel. Le nickel carbonyle convient également bien. On utilise de préférence le nickel RANEY, l'iodure ne nickel, l'acétate de nickel et le nickel carbonyle.

La quantité de nickel n'est pas critique. La teneur en nickel qui a une influence sur la vitesse de réaction est déterminée en fonction de la vitesse de réaction que l'on estime convenable. De manière générale, une quantité comprise entre 5 et 2000 milliatomes-grammes de nickel par litre de solution conduit à des résultats satisfaisants. On opère de préférence avec une teneur comprise entre 20 et 1000 milliatomes-grammes de nickel par litre.

Le procédé selon la présente invention exige également la présence d'au moins un halogénure d'alkyle ou d'acyle. Ces halogénures ont respectivement pour formule $R^2X$ et

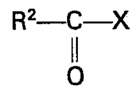

dans lesquelles X représente un atome de chlore, de brome ou, de préférence, un atome d'iode, et $R^2$ a la signification donnée pour $R^1$, $R^1$ et $R^2$ pouvant être identiques ou différents. Bien entendu l'halogénure d'alkyle qui peut être engagé initialement dans le milieu réactionnel, est susceptible d'être formé in situ à partir de dérivés halogénés tels que $Cl_2$, $Br_2$, $I_2$, HCl, HBr, HI, $NiBr_2$ et $NiI_2$, et de l'alcool (matière de départ). En d'autres termes une partie ou la totalité de l'halogénure d'alkyle nécessaire à la mise en oeuvre du présent procédé peut être formé à partir de ses "précurseurs" définis ci-avant.

On constatera en outre que lorsque le dérivé halogéné est choisi parmi les composés du nickel, il peut être considéré comme précurseur non seulement de l'halogénure d'alkyle mais encore comme précurseur du catalyseur métallique. Dans ce cas particulier il s'avère préférable de charger en outre, initialement, un halogénure d'alkyle ou d'acyle et/ou un précurseur distinct des halogénures de nickel en cause.

Dans le cadre de la présente invention, les iodures d'alkyles inférieurs ayant de 1 à 4 atomes de carbone, constituent une classe préférée d'halogénures d'alkyles. L'iodure de méthyle convient particulièrement bien à la mise en oeuvre du procédé selon l'invention.

Pour une bonne mise en oeuvre du présent procédé une concentration en halogénure d'alkyle ou d'acyle d'au moins 0,5 mole par litre de milieu réactionnel est généralement requise. Bien que l'augmentation de la concentration en halogénure d'alkyle ou d'acyle ait un effet faborable sur la vitesse de réaction, il s'avère préférable de ne pas dépasser une concentration de l'ordre de 8 moles par litre. A ce titre une concentration en halogénure d'alkyle ou d'acyle comprise entre 0,8 et 6 moles/litre et de préférence entre 1,5 et 5 moles/litre conduit à des résultats satisfaisants.

L'une des caractéristiques essentielles de la présente invention réside dans la mise en oeuvre d'au moins un sel de chrome, dans lequel le chrome est à un degré d'oxydation (p) supérieur à zéro ($p>0$) c'est-à-dire d'au moins un composé de formule $Cr_n^{p+} X_q^{m-}$ dans laquelle q désigne le rapport $(n \times p)/m$, m est égal à 1, 2 ou 3, $p=2$, 3, 4 ou 6, n étant égal à 1 ou 2, et étant choisi en fonction des valeurs respectives de m et p de telle sorte que q soit un entier, et $X^{m-}$ est un anion choisi dans le groupe comprenant $OH^-$, $Cl^-$, $Br^-$, $I^-$, $O^=$, $PO_4^{\equiv}$, $CH_3COCHC(CH_3)O^-$, $HCOO^-$, $C_2O_4^=$, $R^3$—$COO^-$, $R^3$—$O^-$, $NO_3^-$, $CO_3^=$, $R^3$ ayant la signification donnée pour $R^1$, $R^3$ et $R^1$ pouvant être identiques ou différents; les sels de chrome peuvent être sous forme hydratée.

La nature précise de l'anion $X^{m-}$ ne parait pas être un paramètre fondamental du présent procédé. A titre d'exemples de sels de chrome convenant à la mise en oeuvre du présent procédé on peut citer: $Cr(OH)_3$; $CrCl_2$; $CrCl_3$, $6H_2O$; Cr $Br_2$; Cr $Br_3$, $6H_2O$; $CrO_3$; $Cr_2O_3$; $CrPO_4$, $6H_2O$; Cr $(OCOCH_3)_3$, $H_2O$; $Cr(NO_3)_3$; $CrCO_3$; $Cr(OCOCH_3)_2$; Cr (OH) $(HCO_2)_2$; $Cr[CH_3COCHC(CH_3)O]_3$; $CrC_2O_4$, $H_2O$.

Il n'est pas nécessaire d'engager initialement un sel de chrome, dans lequel le chrome est à un degré d'oxydation supérieur à zéro. En effet, la Demanderesse a constaté qu'il est possible de charger du chrome au degré d'oxydation nul et un oxydant; cet oxydant ne doit pas par ailleurs avoir d'influence néfaste sur la réaction de carbonylation. Ainsi l'espèce active du chrome peut être formée in situ à partir de chrome hexacarbonyle et d'iode. Les carboxylates de chrome et plus particulièrement les acétates sont d'un emploi commode et à ce titre peuvent être préconisés pour la mise en oeuvre de la présente invention. Selon une variante préférée de l'invention on utilise un sel de chrome, dans lequel le chrome est au degré d'oxydation 3 ($p=3$).

De bons résultats sont obtenus lorsque le rapport atomique Cr/Ni est compris entre 0,1 et 200, bien que des rapports inférieurs ou supérieurs puissent être choisis. Ce rapport est aventageusement fixé à une valeur comprise entre 0,5 et 100, et, de préférence, entre 1 et 50.

Lorsqu'on choisit un rapport Cr/Ni faible on peut augmenter l'activité du système catalytique défini ci-avant en ajoutant dans le milieu réactionnel un iodure alcalin, et plus particulièrement un iodure de sodium, de potassium ou de lithium.

Une température de réaction d'au moins 120°C est généralement requise pour obtenir une vitesse de réaction satisfaisante. Une gamme de température comprise entre 160 et 200°C s'avère avantageuse.

Dans le cadre du présent procédé il n'est pas nécessaire de purifier ou sécher l'alcool et l'acide carboxylique engagés initialement à la réaction. On peut utiliser des alcools et des acides carboxyliques de qualité technique, renfermant éventuellement jusqu'à 20 % en volume d'eau. D'autre part on peut engager à la réaction tout ou partie de l'alcool sous forme d'un ester d'un acide $R^1COOH$, $R^1$ ayant la signification donnée précédemment. Dans ce cas on devra également engager initialement de l'eau en quantité au moins égale à la quantité théoriquement nécessaire pour hydrolyser l'ester chargé initialement.

Le procédé de carbonylation selon la présente invention est conduit en phase liquide sous une pression supérieure à la pression atmosphérique, la pression étant toutefois inférieure à 200 bars. On recommande plus particulièrement d'opérer sous une pression partielle de monoxyde de carbone comprise entre 10 et 100 bars. On met en oeuvre, de préférence, du monoxyde de carbone sous forme essentiellement pure, tel qu'il se présente dans le commerce. Toutefois la présence d'impuretés telles que, par exemple du dioxyde de carbone, de l'oxygène, du méthane et de l'azote n'est pas nuisible. La présence d'hydrogène n'est pas nuisible, même en des proportions relativement importantes.

En fin de réaction, on sépare le mélange réactionnel en ses divers constituants par tout moyen approprié, par exemple par distillation.

Le procédé selon l'invention convient particulièrement bien à la préparation de l'acide acétique par carbonylation du méthanol, notamment dans l'acide acétique. Les exemples ci-après illustrent la présente invention sans toutefois en limiter le domaine ou l'esprit.

Dans les exemples les conventions suivantes ont été utilisées:

— AcOH désigne l'acide acétique
— AcOMe désigne l'acétate de méthyle
— RR désigne le rapport molaire

$$\frac{(AcOH+AcOMe)\ dosé-(AcOH)\ initial}{(CH_3OH+CH_3I)\ initial}$$

c'est-à'dire le rapport molaire entre "l'acide acétique potentiel" formé et $(CH_3OH+CH_3I)$ initial.
— v désigne la vitesse initiale de carbonylation exprimée en moles de monoxyde de carbone absorbées par heure et par atome-gramme de nickel
— t désigne la durée effective de l'absorption du monoxyde de carbone à la température de l'essai
— TT désigne le rapport molaire

$$\frac{(CH_3OH)\ initial-[(CH_3OH)+(AcOMe]\ dosés}{(CH_3OH)\ initial}$$

— Pr désigne la productivité, ramenée au temps t (exprimé en heures), en gramme "d'acide acétique potentiel" formé par litre du mélange réactionnel initial

Exemple 1

Dans un autoclave de 125 ml de capacité en Hastelloy B2, on charge:

— 364 mMol de méthanol soit 15 ml
— 341 mMol d'acide acétique soit 20 ml
— 211 mMol d'iodure de méthyle soit 12,5 ml
— 100 mAt-g de chrome sous forme d'acétate de chrome (III), soit 22,9 g
— 20 mMol de nickel tétracarbonyle soit 2,6 ml

Après fermeture de l'autoclave on étabit une pression de 40 bars d'oxyde de carbone. L'agitation par un système de va et vient est mise en route et l'autoclave est porté à 180°C, en 20 minutes environ, au moyen d'un four annulaire. La pression dans l'autoclave est alors de 70 bars; elle est ensuite maintenue égale à 70 bars par des recharges de CO pur.

La chute de pression de la réserve haute pression qui alimente en continu l'autoclave (détendeur) est enregistrée.

L'absorbtion d'oxyde de carbone est terminée après 30 minutes de réaction à 180°C; le chauffage est néanmoins poursuivi pendant encore une heure 30 minutes à cette température.

Après quoi l'agitation et le chauffage sont arrêtés; l'autoclave est refroidi et dégazé.

Le mélange réactionnel résultant est analysé par chromatographie gazeuse, après dilution. Il contient 52,2 g d'acide acétique (RR=92 %). On ne détecte ni acétate de méthyle, ni méthanol.

La productivité (Pr) de la réaction en acide acétique a donc été de 1250 grammes par heure et par litre (g/h×l); le taux de transformation du méthanol est de 100 %.

Exemples 2 à 9

Dans l'appareillage et selon le mode opératoire décrits ci-avant on réalise une série d'essais sur une charge comprenant 15 ml de méthanol, 20 ml d'acide acétique, du nickel, un sel de chrome et de l'iodure de méthyle. La température est de 180°C, la pression est maintenue à 70 bars et la durée de l'essai est de 2 heures (sauf indications contraires). Les conditions particulières ainsi que les résultats obtenus figurent dans le tableau I ci-après.

L'essai témoin (a) figurant également dans le tableau est réalisé en l'absence de sel de chrome.

L'essai témoin (b) figurant également dans ce tableau est réalisé à 190 bars.

Essais témoins (c) à (n):

Dans l'appareillage et selon le mode opératoire décrits pour l'exemple 1 on réalise une série d'essais sur une charge comprenant 15 ml de méthanol, 20 ml d'acide acétique et de l'iodure de méthyle à 180°C et sous 70 bars en présence de divers composés métalliques. *Aucune réaction n'a été observée après 2 heures à la température mentionnée.*

Les conditions particulières de ces essais témoins figurent dans le tableau II ci-après.

TABLEAU I

| No. Ex. | $CH_3I$ mMol | Nickel nature | Nickel mAt-g | Sel de chrome nature | Sel de chrome mAt-g | t (mn) | v | AcOH dosé (g) | AcOMe (g) | TT % | RR % AcOH | Pr g/h×l |
|---|---|---|---|---|---|---|---|---|---|---|---|---|
| 1 | 211 | $Ni(CO)_4$ | 20 | $Cr(OAc)_3$ | 100 | 30 | 30 | 52,2 | 0 | 100 | 92 | 1250 |
| 2 | 207 | ″ | ″ | $CrI_3$ | 40 | 60 | 30 | 36,1 | 0 | 88 | 46 | ND |
| 3 | 208 | $Ni(OAc)_2$ | 8 | $CrO_3$ | 80 | ND | 62,5 | ND | | 95 | ND | ND |
| 4 | 205 | ″ | 10 | { $Cr(CO)_6$ <br> IODE | 30 } <br> 15 } | 120 | 6 | 17,7 | 7,4 | ND | 10 | ND |
| 5 | 206 | ″ | 8 | $Cr(OAc)_3$ | 150 | 40 | 87,5 | 48,7 | 0 | 100 | 86 | 860 |
| 6 | ″ | ″ | ″ | ″ | 50 | 75 | 56 | 45,1 | 0 | 100 | 74 | 410 |
| 7 | 203 | ″ | ″ | ″ | 10 | 120 | 19 | 24,9 | 8,66 | 60 | 34 | 115 |
| 8x | 209 | $Ni(CO)_4$ | 10 | ″ | 10 | ND | 35 | 43,7 | 0 | 100 | 70 | ND |
| 9xx | ″ | ″ | 10 | ″ | ″ | 70 | 45 | 41 | 0 | ″ | 60 | 400 |
| a | 249 | ″ | 20 | ″ | 0 | 120 | 0 | 10,5 | 10,6 | 0 | 0 | 0 |
| b | 199 | $Ni(OAc)_2$ | 8 | $Cr(OAc)_3$ | 100 | ND | ND | 19,4 | 12,3 | 36 | 24 | 80 |

NB: dans ce tableau on a désigné par Ni $(OAc)_2$ l'acétate de nickel tétrahydraté.
ND: non déterminé
x: cet essai est réalisé avec introduction de 100 mMoles de NaI
xx: cet essai est réalisé avec introduction de 50 mMoles de KI

Exemples 10 à 14

Dans l'appareillage et selon le mode opératoire décrits pour l'exemple 1 on réalise une série d'essais sur une charge comprenant du méthanol, de l'acide acétique, de l'iodure de méthyle, 8 mAt-g de nickel sous forme d'acétate de nickel tétrahydraté et 100 mAt-g de chrome sous forme d'acétate de chrome (III). La température est de 180°C; la pression est maintenue à 70 bars (sauf indication contraire) par des recharges de CO pur. La durée de chaque essai est de 2 heures. Les conditions particulières et les résultats obtenus figurent dans le tableau III ci-après.

Exemples 15 à 17

Dans l'appareillage et selon le mode opératoire décrits pour l'exemple 1 on réalise une série d'essais sur une charge comprenant 5 ml d'eau, de l'acétate de méthyle, de l'acide acétique, 8 mAt-g de nickel sous forme d'acétate de nickel tétrahydraté, 80 m At-g de chrome sous forme d'acétate de chrome (III) et de l'iodure de méthyle. La température est de 180°C; la pression totale est maintenue à 70 bars, par des recharges de CO pur. La durée de chaque essai est de 2 heures. Les conditions particulières ainsi que les résultats obtenus figurent dans le tableau IV ci-après.

Exemple 18

Dans l'autoclave et selon le mode opératoire décrits pour l'exemple 1 on charge 15 ml de méthanol, 20 ml d'acide acétique, 162 mMol d'iodure de méthyle, 8 mAt-g de nickel sous forme d'acétate tétrahydraté, et 80 mAt-g de chrome sous forme d'acétate de chrome (III). La température de réaction est de 160°C. La pression totale est maintenue à 70 bars par des recharges de CO pur. En deux heures de réaction à la température indiquée on obtient 24,8 g d'acide acétique et 11,1 g d'acétate de méthyle. (Pr=130 g/h×l).

TABLEAU II
Essais témoins

| Ref. | Nickel | | $CH_3I$ en mMol | Compose metallique | |
|---|---|---|---|---|---|
| | Nature | mAt-g | | Nature | mAt-g |
| c | $Ni(CO)_4$ | 20 | 201 | $Cr(CO)_6$ | 50 |
| d | $Ni(CO)_4$ | 20 | 206 | $Zn(OAc)_2,2H_2O$ | 100 |
| e | $Ni(CO)_4$ | 20 | 203 | Zn | 50 |
| f | $Ni(CO)_4$ | 20 | 209 | $Mn(OAc)_2,4H_2O$ | 100 |
| g | $Ni(CO)_4$ | 20 | 204 | $Al\,I_3$ | 50 |
| h | $Ni(CO)_4$ | 20 | 203 | $Co(OAc)_2,4H_2O$ | 100 |
| i | $Ni(OAc)_2,4H_2O$ | 10 | 205 | $MoBr_3$ | 30 |
| j | $Ni(OAc)_2,4H_2O$ | 8 | 202 | $Fe(OAc)_2$ | 100 |
| k | $Ni(CO)_4$ | 20 | 205 | $MoI_3$ | 10 |
| l | $Ni(CO)_4$ | 10 | 209 | $WI_3$ | 10 |
| m | $Ni(CO)_4$ | 20 | 211 | $Mg(OAc)_2,4H_2O$ | 100 |
| n | $Ni(CO)_4$ | 8 | 200 | $Bi\,I_3$ | 40 |

TABLEAU III

| No. ex. | $CH_3OH$ ml | AcOH ml | $CH_3I$ mMol | TT % | t mn | v | AcOH (g) dosé | AcOMe (g) | RR % | Pr g/h×l |
|---|---|---|---|---|---|---|---|---|---|---|
| 10 | 19 | 26 | 40 | 12 | ND | <6 | 13,4 | 18,4 | 6 | ND |
| 11 | 18 | 24 | 80 | 44 | 120 | 17,5 | 25,9 | 12,9 | 39 | ND |
| 12 | 16 | 22 | 161 | 100 | 75 | 50 | 50,2 | 0 | 85 | 450 |
| 13 | 15 | 20 | 205 | " | 50 | 69 | 46,8 | 0 | 80 | 600 |
| 14x | 15 | " | 204 | 98 | ND | 31 | 48 | 0 | 77 | 270 |

x essai réalisé sous une pression totale de 40 bars

TABLEAU IV

| No. ex. | AcOMe ml | AcOH ml | $CH_3I$ mMol | TT % | t mn | v | AcOH (g) dosé | AcOMe (g) | RR % | Pr g/h×l |
|---|---|---|---|---|---|---|---|---|---|---|
| 15 | 20 | 20 | 84 | 71 | 120 | 19 | 42,6 | 5,4 | 60 | 120 |
| 16 | 10 | 30 | 85 | 100 | 100 | 14 | 49 | 0 | 84 | 125 |
| 17 | 30 | 10 | 84 | 55 | 120 | 20 | 34,8 | 11 | 42 | 110 |

Exemple 19

On reproduit l'exemple 18, mais à une température de 200°C. L'absorption d'oxyde de carbone est terminée après 20 minutes de réaction à la température indiquée; le chauffage est néanmoins poursuivi pendant encore une heure et 40 minutes à cette température. On dose 50,5 g d'acide acétique. Le mélange ne renferme ni acétate de méthyle, ni méthanol. (Pr=1760 g/h×l).

Exemple 20

Dans l'appareillage et selon le mode opératoire décrits pour l'exemple 1 on charge 15 ml de méthanol, 20 ml d'acide acétique, 3,5 ml d'eau, 21 mAt-g de nickel sous forme de nickeltétracarbonyle, 205 mMole d'iodure de méthyle et 100 mAt-g de chrome sous forme d'acétate de chrome (III). La température est de 180°C; la pression totale est maintenue à 70 bars par des recharges de CO pur. La durée de l'essai est de 2 heures en température, bien que l'absorption de monoxyde de carbone soit terminée après 30 minutes.

En fin d'essai on dose 45,5 g d'acide acétique. Le milieu réactionnel ne renferme ni acétale de méthyle, ni méthanol. (Pr=1000 g/h×l).

Exemple 21

Dans l'appareillage et selon le mode opératoire décrits pour l'exemple 1 on charge 25 ml de méthanol, 25 ml d'acide acétique, 8 mAt-g de nickel sous forme d'acétate tétrahydraté, 100 mMol d'iode et 80 mAt-g de chrome sous forme d'acétate de chrome (III). La température est de 180°C. On opère avec une pression partielle d'hydrogène de 30 bars, la pression totale étane maintenue à 90 bars par des recharges de CO pur. La durée de l'essai est de 3 heures. En fin d'essai on dose 55,6 g d'acide acétique. Le mélange réactionnel ne renfreme ni acétate de méthyle, ni méthanol. (RR=83 %).

Exemple 22

Dans un autoclave en titane, de 225 ml de capacité, on charge:

— 755 mMol de méthanol soit 30 ml
— 692 mMol d'acide acétique soit 40 ml
— 415 mMol d'iodure de méthyle soit 25 ml
— 200 mAt-g de chrome sous forme d'acétate de chrome (III) soit 46 g
— 16 mAt-g de nickel sous forme d'acétate de nickel tétrahydraté soit 4 g.

Après fermeture de l'autoclave on établit une pression de 30 bars d'oxyde de carbone. L'agitation par un système de va et vient est mise en route et l'autoclave est porté à 180°C, en 30 minutes environ, au moyen d'un four annulaire. La pression dans l'autoclave est alors de 65 bars; elle est ensuite maintenue égale à 70 bars par des recharges de CO pur.

La chute depression de la réserve haute pression qui alimente en continu l'autoclave (détendeur) est enregistrée.

L'absorbption d'oxyde de carbone est terminée après 40 minutes de réaction à 180°C; le chauffage est néanmoins poursuivi pendant encore 80 minutes.

Après quoi l'agitation et le chauffage sont arrêtés; l'autoclave est refroidi et dégazé

Le mélange réactionnel résultant est analysé par chromatographie gazeuse. Il contient 83,3 g d'acide acétique (RR=60 %). On ne détecte ni méthanol ni acétate de méthyle (TT=100 %).

La productivité de la réaction en acide acétique a donc été de 620 g/h×l. La vitesse initiale de carbonylation a été de 130 moles/h×At-g (Ni).

Exemples 23 à 25

On réalise une série d'essais sur une charge comprenant 30 ml de méthanol, 40 ml d'acide acétique, de l'actate de nickel tétrahydraté (sauf mention contraire), de l'iodure de méthyle et 200 mAt-g de chrome sous forme d'acétate de chrome (III).

Le température est de 180°C; la pression est maintenue à 70 bars par des recharges de CO pur et la durée de chaque essai est de 2 h.

Les conditions particulières ainsi que les résultats obtenus figurent au tableau V ci-après.

L'essai témoin o est réalisé en l'absence de nickel.

TABLEAU V

| No. ex. | Ni mAt-g | $CH_3I$ mMol | t mn | v | TT % | Pr g/h×l |
|---|---|---|---|---|---|---|
| 22 | 16 | 415 | 40 | 130 | 100 | 620 |
| 23 | 8 | " | 90 | 87 | 100 | 250 |
| 24 | 4 | 413 | 120 | 75 | 73 | 160 |
| $25^+$ | 40 | 403 | 25 | 62,5 | 100 | 1400 |
| témoin o | 0 | 420 | — | 0 | 0 | 0 |

(+) essai réalisé avec du nickeltétracarbonyle

**Revendications**

1. Procédé de préparation de l'acide acétique par réaction du monoxyde de carbone sur le méthanol en présence d'une quantité efficace de nickel, d'un halogénure d'alkyle ou d'acyle, d'un acide carboxylique initialement chargé, en phase liquide, à une température supérieure à 120 °C et sous une pression totale inférieure à 200 bars, caractérisé en ce que la réaction est conduite en présence d'au moins un sel de chrome dans lequel le chrome est à un degré d'oxydation (p) supérieur à zéro ($p>0$).

2. Procédé selon la revendication 1, caractérisé en ce que l'halogénure d'alkyle ou d'acyle est un iodure.

3. Procédé selon la revendication 2, caractérisé en ce que l'halogénure d'alkyle est un iodure d'alkyle en $C_1$—$C_4$.

4. Procédé selon la revendication 3, caractérisé en ce que l'iodure d'alkyle est l'iodure de méthyle.

5. Procédé selon l'une quelconque des revendications précédentes, caractérisé en ce que l'acide carboxylique initialement chargé a pour formule $R^1COOH$ dans laquelle $R^1$ représente un radical alkyle linéaire, ramifié ou cyclique en $C_1$—$C_6$ ou un radical $\Phi$—$C_nH_{2n}$—, $1\leqslant n\leqslant 6$.

6. Procédé selon la revendication 5, caractérisé en ce que l'acide carboxylique initialement chargé est l'acide acétique.

7. Procédé selon l'une quelconque des revendications précédentes, caractérisé en ce que les sels de chrome ont pour formule $Cr_n^{p+} X_q^{m-}$ dans laquelle q désigne le rapport $(n\times p)/m$, m est égal à 1, 2 ou 3, $p=2$, 3, 4 ou 6 n étant égal à 1 ou 2 et étant choisi en fonction des valeurs respectives de m et p de telle sorte que q soit un entier, et $X^{m-}$ est un anion choisi dans le groupe comprenant $OH^-$, $Cl^-$, $Br^-$, $I^-$, $O^=$, $PO_4^{\equiv}$, $CH_3COCHC(CH_3)O^-$, $HCOO^-$, $C_2O_4^=$, $R^3$—$COO^-$, $R^3$—$O^-$, $NO_3^-$, $CO_3^=$, $R^3$ ayant la signification donnée pour $R^1$ dans la revendications 5, $R^3$ et $R^1$ pouvant être identiques ou différents.

8. Procédé selon la revendication 7, caractérisé en ce que $p=3$.

9. Procédé selon la revendication 7 ou 8, caractérisé en ce que l'anion ($X^{m-}$) est un carboxylate de formule $R^3$—$COO^-$ dans laquelle $R^3$ a la signification donnée pour $R^1$ dans la revendication 5, $R^3$ et $R^1$ pouvant être identiques ou différents.

10. Procédé selon la revendication 9, caractérisé en ce que l'anion ($X^{m-}$) est un acétate.

11. Procédé selon l'une quelconque des revendications 7 à 10, caractérisé en ce que le rapport atomique Cr/Ni est compris entre 0,1 et 200 et, de préférence, entre 0,5 et 100.

12. Procédé selon la revendication 11, caractérisé en ce que le rapport Cr/Ni est compris entre 1 et 50.

13. Procédé selon l'une quelconque des revendications précédentes, caractérisé en ce que la température de réaction est comprise entre 160 et 200°C.

14. Procédé selon l'une quelconque des revendications précédentes, caractérisé en ce que la pression partielle du monoxyde de carbone est comprise entre 10 et 100 bars.

**Patentansprüche**

1. Verfahren zur Herstellung von Essigsäure durch Umsetzung von Kohlenmonoxid mit Methanol in Gegenwart einer wirksammen Menge Nickel, eines Alkyl- oder Acyl-halogenids, einer ursprünglich aufgegebenen Carbonsäure, in flüssiger Phase, bei einer Temperatur oberhalb 120°C und unter einem Gesamtdruck unter 200 bar, dadurch gekennzeichnet, daß die Reaktion in Gegenwart mindestens eines Chromsalzes ausgeführt wird, in welchem das Chrom einen Oxidationsgrad (p) über 0 aufweist ($p>0$).

2. Verfahren nach Anspruch 1, dadurch gekennzeichnet, daß das Alkyl- oder Acyl-halogenid ein Iodid ist.

3. Verfahren nach Anspruch 2, dadurch gekennzeichnet, daß das Alkyl-halogenid ein $C_1$—$C_4$-Alkyliodid ist.

4. Verfahren nach Anspruch 3, dadurch gekennzeichnet, daß das Alkyliodid Methyliodid ist.

5. Verfahren nach einem der vorangegangenen Ansprüche, dadurch gekennzeichnet, daß die

ursprünglich aufgegebene Carbonsäure der Formel $R^1COOH$ entspricht, in der $R^1$ eine lineare, verzweigte oder cyclische $C_1$—$C_6$-Alkylgruppe oder eine Gruppe $C_6H_5$—$C_nH_{2n}$— mit $1 \leqslant n \leqslant 6$ bedeutet.

6. Verfahren nach Anspurch 5, dadurch gekennzeichnet, daß die ursprünglich aufgegebene Carbonsäure Essigsäure ist.

7. Verfahren nach einem der vorangegangenen Ansprüche, dadurch gekennzeichnet, daß die Chromsalze der Formel $Cr_n^{p+} X_q^{m-}$ entsprechen, in der q das Verhältnis $(n \times p)/m$ bedeutet, wobei m 1, 2 oder 3 ist, p=2, 3, 4 oder 6, n 1 oder 2 ist und in Abhängigkeit von den jeweiligen Werten für m und p gewählt wird, derart daß q eine ganze Zahl ist, und $X^{m-}$ ein Anion aus der Gruppe $OH^-$, $Cl^-$, $Br^-$, $I^-$, $O^=$, $PO_4^{\equiv}$, $CH_3COCHC(CH_3)O^-$, $HCOO^-$, $C_2O_4^=$, $R^3$—$COO^-$, $R^3$- -$O^-$, $NO_3^-$, $CO_3^=$ ist, wobei $R^3$ die im Anspurch 5 für $R^1$ gegebene Bedeutung had und $R^3$ und $R^1$ gleich oder verschieden sein können.

8. Verfahren nach Anspurch 7, dadurch gekennzeichnet, daß p=3 ist.

9. Verfahren nach Anspruch 7 oder 8, dadurch gekennzeichnet, daß das Anion ($X^{m-}$) ein Carboxylat der Formel $R^3$-$COO^-$ ist, in der $R^3$ die für $R^1$ im Anspruch 5 angegebene Bedeutung hat, wobei $R^3$ und $R^1$ gleich oder verschieden sein können.

10. Verfahren nach Anspruch 9, dadurch gekennzeichnet, daß das Anion ($X^{m-}$) das Acetatanion ist.

11. Verfahren nach einem der Ansprüche 7 bis 10, dadurch gekennzeichnet, daß das Atomverhältnis Cr/Ni 0,1 bis 200 und vorzugsweise 0,5 bis 100 beträgt.

12. Verfahren nach Anspruch 11, dadurch gekennzeichnet, daß das Verhältnis Cr/Ni 1 bis 50 beträgt.

13. Verfahren nach einem der vorangegangenen Ansprüche, dadurch gekennzeichnet, daß die Reaktionstemperatur im Bereich von 160 bis 200°C liegt.

14. Verfahren nach einem der vorangegangenen Ansprüche, dadurch gekennzeichnet, daß der Partialdruck des Kohlenmonoxids 10 bis 100 bar beträgt.

**Claims**

1. Process for the preparation of acetic acid by reacting carbon monoxide with methanol in the presence of an effective amount of nickel, an alkyl or acyl halide and a carboxylic acid initially introduced, in the liquid phase, at a temperature above 120°C and under a total pressure below 200 bars, characterised in that the reaction is called out in the presence of at least one chromium salt in which the chromium is in an oxidation state (p) above zero (p>0).

2. Process according to claim 1, characterised in that the alkyl or acyl halide is an iodide.

3. Process according to claim 2, characterised in that the alkyl halide is a $C_1$—$C_4$ alkyl iodide.

4. Process according to claim 3, characterised in that the alkyl iodide is methyl iodide.

5. Process according to any one of the preceding claims, characterised in that the carboxylic acid initially introduced has the formula $R^1COOH$, in which $R^1$ represents a linear, branched or cyclic $C_1$—$C_6$ alkyl radical or a radical $\Phi$—$C_nH_{2n}$—, $1 \leqslant n \leqslant 6$.

6. Process according to claim 5, characterised in that the carboxylic acid initially introduced is acetic acid.

7. Process according to any of the preceding claims, characterised in that the chromium salts have the formula $Cr_n^{p+} X_q^{m-}$, in which q denotes the ratio $(n \times p)/m$, m is equal to 1, 2, or 3, p=2, 3, 4 or 6, n being equal to 1 or 2 and being chosen according to the respective values of m and p such that q is an integer, and $X^{m-}$ is an anion chosen from the group comprising $OH^-$, $Cl^-$, $Br^-$, $I^-$, $O^=$, $PO_4^{\equiv}$, $CH_3COCHC(CH_3)O^-$, $HCOO^-$, $C_2O_4^=$, $R^3$—$COO^-$, $R^3$—$O^-$, $NO_3^-$ and $CO_3^=$, $R^3$ having the meaning given for $R^1$ in claim 5, and it being possible for $R^3$ and $R^1$ to be identical or different.

8. Process according to claim 7, characterised in that p=3.

9. Process according to claim 7 or 8, characterised in that the anion ($X^{m-}$) is a carboxylate of the formula $R^3$—$COO^-$, in which $R^3$ has the meaning given for $R^1$ in claim 5, it being possible for $R^3$ and $R^1$ to be identical or different.

10. Process according to claim 9, characterised in that the anion ($X^{m-}$) is an acetate.

11. Process according to any one of claims 7 to 10, characterised in that the atomic ratio Cr/Ni is from 0.1 to 200 and preferably from 0.5 to 100.

12. Process according to claim 11, characterised in that the ratio Cr/Ni is from 1 to 50.

13. Process according to any of the preceding claims, characterised in that the reaction temperature is from 160 to 200°C.

14. Process according to any of the preceding claims, characterised in that the partial pressure of the carbon monoxide is from 10 to 100 bars.